**Europäisches Patentamt**

**European Patent Office**

**Office européen des brevets**

(11) Veröffentlichungsnummer: **0 415 334 A1**

## (12) EUROPÄISCHE PATENTANMELDUNG

(21) Anmeldenummer: 90116421.0

(22) Anmeldetag: 28.08.90

(51) Int. Cl.⁵: **C07C 41/58**, C07C 43/15

(30) Priorität: 31.08.89 DE 3928774

(43) Veröffentlichungstag der Anmeldung:
06.03.91 Patentblatt 91/10

(84) Benannte Vertragsstaaten:
AT BE DE FR GB IT NL SE

(71) Anmelder: HOECHST AKTIENGESELLSCHAFT
Postfach 80 03 20
W-6230 Frankfurt am Main 80(DE)

(72) Erfinder: Mildenberger, Hilmar, Dr.
Fasanenstrasse 24
W-6233 Kelkheim/Taunus(DE)
Erfinder: Lachhein, Stephen, Dr.
Kiedricher Strasse 18
W-6238 Hofheim am Taunus(DE)
Erfinder: Rittner, Siegbert, Dr.
Kornblumenweg 5
W-6082 Mörfelden(DE)
Erfinder: Tetzlaff, Heribert, Dr.
Geisenheimer Strasse 88
W-6000 Frankfurt am Main(DE)

(54) Verfahren zur Abtrennung von Enolethern aus Reaktionsgemischen mit Alkoholen.

(57) Die Erfindung betrifft ein schonendes Verfahren zur extraktiven Trennung von Enolether aus Gemischen mit wasserlöslichen Alkoholen, wobei als Extraktionsmittel eine wäßrige Lösung einer Base in einem Phasenmengenverhältnis wäßrige Phase : Enolether/Alkohol-Gemisch von mindestens 0,5 : 1 verwendet und die Extraktion mehrstufig oder kontinuierlich mit einer theoretischen Stufenzahl von mindestens zwei durchgeführt wird. Das Verfahren eignet sich besonders zur Abtrennung von Enolethern, die sich bei der Destillation leicht zersetzen.

# VERFAHREN ZUR ABTRENNUNG VON ENOLETHERN AUS REAKTIONSGEMISCHEN MIT ALKOHOLEN

Enolether sind wichtige Zwischenverbindungen zur Synthese cyclischer und heterocyclischer Verbindungen (z. B. Pflanzenschutzmittel) oder auch zur Darstellung von Allylphenolen (durch Claisen-Umlagerung aus Allylphenylethern). Bei ihrer Darstellung aus Ketonen und beispielsweise Methanol fallen sie im Gemisch mit Methanol an. Die Abtrennung von Enolethern aus methanolischer Lösung durch Destillation bzw. Rektifikation bei erniedrigtem Druck ist beispielsweise in JP-A 75/96512 und J. Org. Chem. 40 (1981), S. 2532 ff. beschrieben.

Falls Methanol und Enolether ein Azeotrop bilden, kann in manchen Fällen die Extraktivdestillation eingesetzt werden, bei der eine zugesetzte, hochsiedende dritte Komponente zusammen mit einem der beiden Stoffe im Kolonnensumpf verbleibt (DE-C 2 305 021, L. Berg, A. Yeh in AIChE Journal 31 (1985), Seite 504 ff). Die Destillationsverfahren haben den Nachteil, daß empfindliche Enolether dabei teilweise oder ganz zersetzt werden.

Es ist bekannt, daß man in manchen Fällen bei der destillativen Reinigung von Methanol/Enolether-Gemischen im Labor einen Teil des Methanols vor der Destillation durch Waschen mit verdünnter KOH-Lösung entfernen kann (J. Chem. Thermodynamics 5 (1973), 783 ff.). Die noch erforderliche destillative Trennung bei dem bekannten Verfahren ist aus den obengenannten Gründen jedoch weiterhin nachteilig.

Es bestand daher die Aufgabe, in Verfahren zur Trennung von Enolether/Alkohol-Gemischen bereitzustellen, das für empfindliche Enolether geeignet ist und auch großtechnisch durchgeführt werden kann.

Gegenstand der Erfindung ist ein Verfahren zur extraktiven Trennung von Enolethern und wasserlöslichen Alkoholen, dadurch gekennzeichnet, daß als Extraktionsmittel eine wäßrige Lösung einer Base in einem Phasenmengenverhältnis wäßrige Phase : Enolether/Alkohol-Gemisch von mindestens 0,5:1, vorzugsweise mindestens 1:1, insbesondere von 1,4:1 bis 1,7:1, bezogen auf die Gewichte der Phasen verwendet, und die Extraktion mehrstufig oder kontinuierlich mit einer theoretischen Stufenzahl von mindestens 2 durchgeführt wird.

Als Enolether eignen sich alle weitgehend wasserunlöslichen und unterhalb der kritischen Temperatur des Systems Enolether-Alkohol-Wasser (Verschwinden der Mischungslücke) flüssigen Enolether.

Vorzugsweise eignen sich Enolether aus der Gruppe der $2\text{-}(C_1\text{-}C_4\text{-Alkyloxy})\text{-}C_3\text{-}C_{16}\text{-alk-1-ene}$, wie 2-Methoxy-, 2-Ethoxy-, 2-Propoxy-, 2-Isopropoxy-, 2-n-Butoxy-, 2-Isobutoxy-, 2-t-Butoxy-, 2-sec-Butoxy-alkene, insbesondere 2-Methoxy-3-methyl-1-buten.

Als wasserlösliche Alkohole eignen sich vor allem Methanol, Ethanol, Propanol, Isopropanol, n-Butanol, Isobutanol, insbesondere aber Methanol. Bevorzugt werden zur Trennung Enolether/Alkohol-Gemische eingesetzt, wie sie bei der Herstellung von Enolethern aus den entsprechenden Ketalen durch Abspaltung von Alkohol und der nachfolgenden Aufarbeitung entstehen, insbesondere azeotrope Gemische aus Enolether und Alkohol.

Für das erfindungsgemäße Verfahren werden als Extraktionsmittel zweckmäßig wäßrige Lösungen von anorganischen und/oder organischen Basen eingesetzt. Die Lösungen haben in der Regel einen pH-Wert zwischen 7 und 14. Bevorzugt sind verdünnte, schwach basische Lösungen mit einem pH-Wert zwischen 7 und 12. Die Normalitäten liegen dabei in der Regel im Bereich von $10^{-4}$ bis 1 N, vorzugsweise $10^{-4}$ bis $10^{-1}$ N, Basenlösung.

Geeignete basische Extraktionsmittel sind verdünnte Lösungen von Alkalimetallhydroxiden wie NaOH und KOH, löslichen Carbonaten, wie Alkalimetallcarbonaten (z. B. $Na_2CO_3$, $K_2CO_3$) oder $(NH_4)_2CO_3$, und Erdalkalimetallhydroxiden wie $Ba(OH)_2$ und $Ca(OH)_2$. Ebenfalls geeignet sind Lösungen organischer Basen wie Amine, z. B. tertiäre Amine wie Triethylamin, Trimethylamin, Ethyldimethylamin. Bevorzugt sind 0,0005 bis 1 N, insbesondere 0,0005 bis 0,002 N NaOH-oder KOH-Lösungen sowie 0,005 bis 1 N, insbesondere 0,0005 bis 0,02 normale wäßrige Lösungen von $Na_2CO_3$, $K_2CO_3$ oder Triethylamin.

Das erfindungsgemäße Verfahren wird beispielsweise so durchgeführt, daß das zu trennende Enolether/Alkohol-Gemisch mit der geeigneten Menge an Extraktionsmittel innig vermischt und dann bis zur Phasentrennung stehengelassen wird. Nach erfolgter Abtrennung der Phasen mit Hilfe üblicher Vorrichtungen wird die Prozedur mit der Enoletherphase ein- oder mehrmals bis zur gewünschten Reinheit wiederholt. Um Verluste an Enolether über die wäßrige Phase zu vermeiden, wird die mehrstufige Extraktion bevorzugt nach dem Gegenstromprinzip durchgeführt. Das mehrstufige Verfahren kann diskontinuierlich, halbkontinuierlich oder kontinuierlich erfolgen. Schon nach zwei Trennvorgängen können in Abhängigkeit vom jeweiligen Enolether, dem Phasenmengenverhältnis und der Temperatur Reinheiten von über 99,8 Gew.-% in der Enoletherphase erreicht werden. Die Temperatur ist aus praktischen Gründen vorzugsweise bei 10 bis 30° C, insbesondere bei Raumtemperatur, kann aber prinzipiell zwischen dem Schmelzpunkt der Mischung und dem kritischen Punkt (Verschwinden der Mischungslücke) liegen, wobei die Trennung bei tieferen Temperaturen in der Regel etwas besser wird.

Die Güte der Trennung zeigen die Verteilungskoeffizienten $K_D$, hier definiert als das Verhältnis der Konzentration eines Stoffes in der Enoletherphase zu seiner Konzentration in der wäßrigen Phase. Der nötige Trennaufwand zur Spaltung der Enolether/Alkohol-Gemische wird durch den Trennfaktor $\beta = K_D$ - (Enolether)/$K_D$ (Alkohol) charakterisiert. Er ist ein Maß für die benötigte Stufenzahl und das Phasenmengenverhältnis bei der Realisierung eines Extraktions-Trennverfahrens.

Die extraktive Trennung von Enolether/Alkohol-Gemischen erfolgt am wirtschaftlichsten durch mehrstufige kontinuierliche Gegenstromverteilung. Als Apparate zur Durchführung der Extraktion sind alle gängigen Extraktionsapparate geeignet (z. B. Kolonnen, Mischabsetzer, Graesser-Extraktor, Zentrifugalextraktor).

Ein weiterer Vorteil des erfindungsgemäßen schonenden Extraktions-Trennverfahrens liegt darin, daß der Grad der Abtrennung des Alkohols aus der Enoletherphase über die Stufenzahl beliebig hoch gewählt werden kann. Die schwach basische wäßrige Lösung verhindert außerdem eine Zersetzung des Enolethers zum Ketal.

Geringe Reste (weniger als 0,5 Gew.-%) an Enolether, die mit der wäßrig-alkoholischen Raffinatphase aus der Extraktion ablaufen, können daraus destillativ als Alkohol/Enolether-Azeotrop abgetrennt und wieder in die Extraktionsstufe des Verfahrens zurückgeführt werden.

Bei der destillativen Aufarbeitung kann außerdem neben dem Alkohol/Enolether-Azeotrop die Hauptmenge an extrahiertem Alkohol von der wäßrigen Phase abgetrennt werden, wobei die so gereinigte wäßrige Phase im Kreislauf zurückgeführt, d. h. als Extrationsmittel für das erfindungsgemäße Verfahren wieder eingesetzt werden kann. Mit destillativer Aufarbeitung der wäßrig-alkoholischen Phase, Rückführung des Enolether/Alkohol-Azeotrops und Rückführung der wäßrigen, basischen Phase liefert das Verfahren nur die im Ausgangsgemisch enthaltenen, nunmehr getrennten Komponenten Enolether und Alkohol, wobei der Reinheitsgrad des Enolethers mit mehrstufiger Verfahrensweise im Gegenstromprinzip beliebig gesteigert werden kann.

Die destillative Aufarbeitung der wäßrig-alkoholischen Phase zur Rückführung der Enoletherreste eignet sich vor allem bei Methanol als Alkohol. Bei anderen wasserlöslichen Alkoholen erschwert manchmal die Azeotropbildung Wasser/Alkohol die destillative Aufarbeitung des wäßrig-alkholischen Raffinats und damit einen Kreislauf-Wiedereinsatz (Rückführung) der wäßrigen Phase.

Bei Alkoholen, deren Siedepunkte dabei über 100 °C liegen, sollten salzartige Basen zur Herstellung des basischen Milieus in der wäßrigen Phase vermieden werden, da diese bei der destillativen Raffinataufarbeitung in der Alkoholphase abgeschieden werden können. In solchen Fällen sind flüssige organische Basen besser geeignet.

Das Verfahren wird in den folgenden Beispielen 1 bis 4 näher erläutert, ohne daß die möglichen Verfahrensbedingungen auf die gezeigten Beispiele beschränkt sein sollen:

1. Trennung eines Gemisches von 2-Methoxy-3-methyl-1-buten (51,07 Gew.-%) und Methanol (48,93 Gew.-%): Durch dreistufige schubweise Gegenstromextraktion nach dem Schema von Watanabe und Morikawa (siehe z.B. O. Jübermann in HOUBEN WEYL, Methoden der organischen Chemie, 4. Aufl. (1958), G. Thieme Verlag Stuttgart, Bd. I/1 S. 255 ff.) bei Raumtemperatur und beim Phasenmengenverhältnis Enolether/Methanol-Gemisch zu 0,001 N NaOH von 1 : 1,45 wird ein Gemisch aus 51,07 Gew.-% 2-Methoxy-3-methyl-1-buten (I) und 48,93 Gew.-% Methanol in eine Enoletherphase mit 99,83 Gew.-% (I), höchstens 0,02 Gew.-% Methanol und 0,15 Gew.-% Wasser und in eine wäßrige Phase mit 25,18 Gew.-% Methanol und 0,41 Gew.-% (I) getrennt. Die Mengenbilanz zeigt Tabelle 1 (siehe Seite 6).

2. Es wird ein Gemisch wie in Beispiel 1 beschrieben eingesetzt. Durch vierstufige kontinuierliche Gegenstromextraktion in einer Labormischabsetzerbatterie bei Raumtemperatur und beim Phasenmengenverhältnis Enolether/Methanol-Gemisch zu 0,01 N $Na_2CO_3$-Lösung von

Tabelle 1:

| 3-stufige Gegenstromextraktion eines Enolether/Methanol-Gemisches (S) mit 0,001 N NaOH-Lösung (X) bei Raumtemperatur. | | | | |
|---|---|---|---|---|
| Die Extraktion wird schubweise nach dem Schema von Watanabe-Morikawa durchgeführt, was die gleichen Ergebnisse wie eine kontinuierliche Gegenstromextraktion bei 100 %iger Stufenwirksamkeit ergibt. Phasenmengenverhältnis: S : X = 1 : 1,45. | | | | |
| | Menge | Enolether | Methanol | Wasser |
| | g | (Gew.-%) | (Gew.-%) | (Gew.-%) |
| Speiselösung (S) | 136,88 | 51,07 | 48,93 | -- |
| 0,001 N NaOH | 197,99 | -- | -- | ca. 100 |
| Enoletherphase | 68,93 | 99,83 | ≦ 0,02 | 0,15 |
| wäßrige Phase | 265,94 | 0,41 | 25,18 | 74,41 |

1 : 1,7 wird ein Gemisch aus 57,8 Gew.-% (I) und 42,2 Gew.-% Methanol in eine Enoletherphase mit 99,91 Gew.% (I), höchstens 0,01 Gew.-% Methanol und 0,08 Gew.-% Wasser und in eine wäßrige Phase mit 19,7 Gew.-% Methanol und 0,39 Gew.-% (I) getrennt.

3. Es wird ein Gemisch wie in Beispiel 1 beschrieben eingesetzt. Durch kontinuierliche Gegenstromextraktion in einer Labor-Karr-Kolonne (Schwingbodenkolonne mit Durchmesser 25 mm und Höhe 2 m, theoretische Stufenzahl 5) bei Raumtemperatur und beim Phasenmengenverhältnis Enolether/Methanol-Gemisch zu 0,01 N wäßriger Triethylaminlösung von 1 : 1,5 wird ein Gemisch aus 52,3 Gew.-% (I) und 47,7 Gew.-% Methanol in eine Enoletherphase mit 99,89 Gew.-% (I), 0,02 Gew.-% Methanol und 0,09 Gew.-% Wasser sowie in eine wäßrige Phase mit 24,0 Gew.-% Methanol und 0,45 Gew.-% (I) getrennt. Das Triethylamin gelangt dabei z. T. in die Enoletherphase und stabilisiert den Enolether zusätzlich gegen Zersetzung.

4. Es wird ein Gemisch aus 81,5 Gew.-% Enolether und 18,5 Gew.-% Methanol eingesetzt (10 kg/h). Durch kontinuierliche Gegenstromextraktion in einem 4-stufigen Zentrifugalextraktor (Typ LX 124, Fa. Robatel, Drehzahl 2900 min⁻¹) mit 0,01 N Natronlauge (10 kg/h) wird ein mehr als 99,93 gew.-%iger Enoletherextrakt gewonnen, der weniger als 0,07 Gew.-% Methanol enthält.

**Vergleichsbeispiele A, B, C:**

Anhand der Verteilungskoeffizienten und Trennfaktoren wird die Wirksamkeit des Extraktions-Trennverfahrens zur Aufspaltung von Enolether/Methanol-Gemischen aufgezeigt. Dazu wird jeweils eine bestimmte Menge einer etwa hälftigen Mischung aus 2-Methoxy-3-methyl-1-buten und Methanol mit dem Extraktionsmittel (0,001 N NaOH) durch intensives Vermischen bei Raumtemperatur ins Gleichgewicht gesetzt. Dann läßt man die Phasen absitzen, trennt sie und bestimmt die Konzentrationen der Bestandteile in beiden Phasen. In Tabelle 2 sind die Versuchsergebnisse der Vergleichsbeispiele zusammengefaßt.

Die Vergleichsbeispiele A, B und C zeigen, daß bei einer einstufigen Verfahrensweise auch unter starker Erhöhung des Phasenverhältnisses wäßrige Phase : Enolether/Alkohol-Gemisch keine ausreichende Abtrennung des Alkohols aus der Enoletherphase möglich ist.

Tabelle 2:

| Mengenbilanz, Verteilungskoeffizienten $K_D$ ( = Konzentration eines Stoffes in der Enoletherphase/Konzentration dieses Stoffes in der wäßrigen Phase) und Trennfaktoren $\beta$ ( = $K_D$ (Enolether)/$K_D$ (Methanol)) bei der Aufspaltung von Enolether/Methanol-Mischungen mit 0,001 N NaOH; S = Speiselösung (eingesetztes Gemisch) | | | | | | | | |
|---|---|---|---|---|---|---|---|---|
| | Menge g | Enolether (Gew.-%) | Methanol (Gew.-%) | Wasser (Gew.-%) | $K_D$ (Enolether) | $K_D$ (Methanol) | $K_D$ (Wasser) | $\beta$ |
| **A)** | | | | | | | | |
| Einwaage S | 39,80 | 50,00 | 50,00 | -- | | | | |
| Einwaage 0,001 N NaOH | 49,83 | -- | -- | ca. 100 | | | | |
| Enoletherphase | 19,76 | 99,14 | 0,63 | 0,23 | | | | |
| wäßrige Phase | 69,87 | 0,44 | 28,30 | 71,26 | 230 | 0,022 | 0,0032 | 10 000 |
| **B)** | | | | | | | | |
| Einwaage S | 19,74 | 50,00 | 50,00 | -- | | | | |
| Einwaage 0,001 N NaOH | 49,80 | -- | -- | ca. 100 | | | | |
| Enoletherphase | 9,78 | 99,54 | 0,28 | 0,18 | | | | |
| wäßrige Phase | 59,76 | 0,23 | 16,47 | 83,30 | 430 | 0,017 | 0,0022 | 25 000 |
| **C)** | | | | | | | | |
| Einwaage S | 15,75 | 50,00 | 50,00 | -- | | | | |
| Einwaage 0,001 N NaOH | 79,50 | -- | -- | ca. 100 | | | | |
| Enoletherphase | 7,77 | 99,72 | 0,14 | 0,14 | | | | |
| wäßrige Phase | 87,48 | 0,15 | 8,99 | 90,86 | 660 | 0,016 | 0,0015 | 41 000 |

## Ansprüche

1. Verfahren zur extraktiven Trennung von Enolethern und wasserlöslichen Alkoholen, dadurch gekennzeichnet, daß als Extraktionsmittel eine wäßrige Lösung einer Base in einem Phasenmengenverhältnis wäßrige Phase : Enolether/Alkohol-Gemisch von mindestens 0,5:1, bezogen auf die Gewichte der Phasen, verwendet und die Extraktion mehrstufig oder kontinuierlich mit einer theoretischen Stufenzahl von mindestens 2 durchgeführt wird.

2. Verfahren nach Anspruch 1, dadurch gekennzeichnet, daß ein Gemisch aus einem 2-($C_1$-$C_4$-Alkyloxy)-$C_3$-$C_9$-alk-1-en und einem $C_1$-$C_4$-Alkohol eingesetzt wird.

3. Verfahren nach Anspruch 1 oder 2, dadurch gekennzeichnet, daß ein azeotropes Gemisch aus Enolether und Alkohol eingesetzt wird.

4. Verfahren nach einem oder mehreren der Ansprüche 1 bis 3, dadurch gekennzeichnet, daß das Phasenmengenverhältnis wäßrige Phase : Enolether/Alkohol-Gemisch von 1,4:1 bis 1,7:1 beträgt.

5. Verfahren nach einem oder mehreren der Ansprüche 1 bis 4, dadurch gekennzeichnet, daß als wäßrige Lösung einer Base eine verdünnte wäßrige Lösung von NaOH, KOH, Ba(OH)$_2$, Ca(OH)$_2$ oder einem Amin eingesetzt wird.

6. Verfahren nach einem oder mehreren der Ansprüche 1 bis 5, dadurch gekennzeichnet, daß der pH-Wert in der wäßrigen Phase zwischen 7 und 14 ist.

7. Verfahren nach einem oder mehreren der Ansprüche 1 bis 6, dadurch gekennzeichnet, daß die Extraktion mehrstufig nach dem Gegenstromprinzip durchgeführt wird.

8. Verfahren nach einem oder mehreren der Ansprüche 1 bis 7, dadurch gekennzeichnet, daß die Temperatur bei 10 bis 30° C ist.

9. Verfahren nach einem oder mehreren der Ansprüche 1 bis 8, dadurch gekennzeichnet, daß die nach der Extraktion auftretende wäßrig-alkoholische Raffinatphase destillativ aufgearbeitet wird und die dabei als Alkohol/Enolether-Azeotrop abgetrennten Reste an Enolether und die von der Hauptmenge an Alkohol befreite wäßrige Phase in die Extraktion zurückgeführt werden.

10. Verfahren nach einem oder mehreren der Ansprüche 1 bis 9, dadurch gekennzeichnet, daß der wasserlösliche Alkohol Methanol ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | DE - C - 863 940 (THE BRITISH OXYGEN COMP.) * Ansprüche 1,3; Beispiele 1 * -- | 1-3 | C 07 C 41/58 C 07 C 43/15 |
| X | DE - C - 841 457 (THE BRITISH OXYGEN COMP.) * Ansprüche 1,3,4; Beispiel 1 * -- | 1-3 | |
| D,A | DE - C - 2 305 021 (KYOWO YUKA) * Anspruch 1 * ---- | 1 | |

**RECHERCHIERTE SACHGEBIETE (Int Cl⁵)**

C 07 C 41/00
C 07 C 43/00
C 07 C 29/00

Der vorliegende Recherchenbericht wurde für alle Patentansprüche erstellt

| Recherchenort | Abschlußdatum der Recherche | Prüfer |
|---|---|---|
| WIEN | 07-12-1990 | REIF |